# EUROPÄISCHE PATENTSCHRIFT

(11) **EP 2 237 752 B1**
(45) Veröffentlichungstag und Bekanntmachung des Hinweises auf die Patenterteilung: **05.12.2012**
(21) Anmeldenummer: 09703625.5
(22) Anmeldetag: 26.01.2009
(51) Int. Cl.: A61F 7/00, A41D 19/015

(54) **HEIZVORRICHTUNG FÜR EINEN HANDSCHUH**
HEATING DEVICE FOR A GLOVE
SYSTÈME DE CHAUFFAGE POUR UN GANT

(30) Priorität: 25.01.2008 DE 102008006939
(43) Veröffentlichungstag der Anmeldung: 13.10.2010
(73) Patentinhaber: Therm-IC Products GmbH, 8200 Gleisdorf (AT)
(72) Erfinder: MACHER, David, 8570 Voitsberg (AT); KREMER, Gerhard, A-8280 Fürstenfeld (AT); MARON, Urs, A-8144 Haselsdorf-Tobelbad (AT)
(74) Vertreter: Pfenning, Meinig & Partner GbR
(86) Internationale Anmeldenummer: PCT/EP2009/000596
(87) Internationale Veröffentlichungsnummer: WO 2009/092618

(56) Entgegenhaltungen:
- DE-A1- 2 903 898
- FR-A- 2 645 410
- JP-A- 4 098 718
- US-A- 3 708 649
- US-A1- 2003 232 518
- US-B1- 6 239 410
- US-B1- 6 649 873
- US-B1- 7 307 242

## Beschreibung

Gegenstand der Erfindung ist eine Heizvorrichtung nach den Merkmalen des Oberbegriffs des Anspruchs 1.

Heizeinrichtungen, insbesondere zum Erwärmen und/oder Temperieren einer Hautoberfläche mit Tiefenwirkung auf Teilbereiche eines menschlichen Körpers mit mindestens einem Wärmeübertragungselement und mindestens einem Anschluss für eine Energieversorgungseinrichtung sowie einem Bedienfeld mit einer Umkapselung sind im Stand der Technik bekannt. So zeigt beispielsweise die Druckschrift DE 198 08 851 A1 eine derartige Heizeinrichtung.

Nachteil an der darin gezeigten Heizeinrichtung ist es, dass das Bedienfeld nur schwierig in einem Handschuh oder in einem Kleidungsstück einsetzbar ist, da es zum einen eine zu große Raumausdehnung besitzt und das Bedienfeld in einer außen am z. B. Handschuh befestigten Tasche angeordnet sein muss. Dadurch verschlechtert unter anderem auch die Bedienbarkeit, da ein Träger der Handschuhe die Tasche erst öffnen muss und erst anschließend auf das Bedienfeld zugreifen kann.

Eine feste Positionierung des Bedienfelds ist nicht möglich.

Ein weiteres Beispiel für ein Kleidungsstück, in diesem Falle ein Handschuh, mit einer Heizeinrichtung ist beispielsweise in der US 2005/0155961 A1 gezeigt. Auch hierbei ist das Bedienfeld hinter einem Klettverschluss versteckt.

Aus der "Elektronik 22/2003, S. 52-57" ist ein Kleidungsstück mit einem integrierten Bedienfeld bekannt. Allerdings wird hier eine direkte Vernähung zwischen der Elektronik und dem Kleidungsstück vorgenommen, so dass erst beim vollständigen Anfertigen des Kleidungsstücks getestet werden kann, ob das Bedienfeld funktioniert. Ist dies nicht der Fall, muss das Kleidungsstück wieder aufgetrennt werden, was mit hohen Mehrkosten verbunden ist.

Ein weiteres Beispiel für einen mit einem Kontrollpaneel verbundenen Handschuh kann aus der US 2006/0092624 A1 entnommen werden. Nachteilig ist hier jedoch, dass das Bedienfeld nicht integral mit dem Handschuh verbunden ist und hierdurch ein stärkerer Fokus auf den Verbindungsmechanismus gelegt werden muss.

US 7,307,242 offenbart eine Heizeinrichtung für einem Handschuh nach dem Oberbegriff des Anspruchs 1.

Aufgabe der vorliegenden Erfindung ist es, eine Heizeinrichtung zu schaffen, welche einfach und komfortabel bedienbar ist, von geringer Baugröße sein kann und einfach in ein Kleidungsstück oder dergleichen einsetzbar ist. Die Aufgabe wird gelöst mit einen Heizvorrichtung mit den Merkmalen des Anspruchs 1. Vorteilhafte Weiterbildungen sind in den abhängigen Ansprüchen beschrieben.

Da die Heizeinrichtung des Kleidungsstücks eine Umkapselung mit einer die Leiterplatte aufnehmenden Kammer und einen die Kammer umgebenden flanschförmigen Rand aufweist, wird zum einen die Leiterplatte mit der darauf befindlichen Elektronik und Elektrik geschützt. Dabei wird die Leiterplatte innerhalb der Kammer angeordnet und kann aus der Kammer aufgrund des vorhandenen zumindest teilweise geschlossenen Randes nicht außerhalb der Kammer rutschen. Mit dem die Kammer umgebenden flanschförmigen Rand kann eine derartige Heizeinrichtung einfach in ein Kleidungsstück integriert werden. Dabei wird der flanschförmige Rand, welcher aus einem flexiblen Material besteht, mit der Außenhülle des Kleidungsstücks verbunden. Eine zusätzliche Tasche zur Aufnahme des Bedienfelds ist dadurch nicht vonnöten. Des Weiteren ist das Bedienfeld kraftschlüssig mit dem Kleidungsstück verbindbar, ohne die Leiterplatte zu gefährden, da diese alleinig in der Kammer angeordnet ist.

Dadurch, dass die zwei Schalter zum manuellen Einstellen der Temperatur, einer zum Erhöhen und einer zur Verringerung der Temperatur, durch jeweils ein bzw. zwei auf einer Innenseite der Umkapselung angeordnete(s) Kontaktelement(e) und ein bzw. zwei auf der Leiterplatte ausgebildete Kontaktelementen gebildet sind, können die Schalter Platz sparend aus einer Kombination der Umkapselung und der Leiterplatte geschaffen werden.

Dadurch ist keine weitere Verdrahtung zwischen einem Schalter und der Leiterplatte notwendig: die Leiterplatte wird Teil des Schalters. Des Weiteren ist es dadurch möglich, das Bedienfeld sehr klein auszubilden, was die Integration in das Kleidungsstück, wie z. B. einen Handschuh, zusätzlich vereinfacht.

Die Umkapselung besteht aus siliken. Hierdurch ist die Umkapselung wasserdicht ausführbar, so dass die in der Kammer angeordnete Leiterplatte optimal geschützt wird.

Die Heizeinrichtung wird bevorzugt unabhängig vom Kleidungsstück und vor dem Verbinden mit demselbigen hergestellt und getestet. Anschließend wird die Umkapselung beispielsweise derart in einer Aussparung des Kleidungsstücks angeordnet, dass die Kammer vorzugsweise direkt zugängig in der Aussparung liegt und der flanschförmige Rand mit dem Rand der Aussparung verbunden wird, beispielsweise durch Vernähen oder Verkleben.

Dadurch, dass die Verbindung zwischen der Umkapselung und dem Kleidungsstück über den flanschförmigen Rand hergestellt wird, besteht keine Gefahr, dass die Leiterplatte beim Verbinden beschädigt wird.

Nachfolgend wird die Erfindung am Beispiel eines Handschuhs erläutert, wobei die Kleidungsstücke auch eine Jacke, eine Hose oder Strümpfe sein können.

Ein erfindungsgemäßer Handschuh weist eine Außenhülle und eine Heizvorrichtung, wie vorbeschrieben, auf, wobei der flanschförmige Rand der Umkapselung im Bereich eines Handrückens mit der Außenhülle verbunden, vorzugsweise vernäht ist. Dadurch, dass die Leiterplatte lediglich ein geringes Ausmaß vorweisen muss (beispielsweise eine Fläche zwischen 1 bis 8 cm²) und vor Schlägen oder Verbiegungen geschützt werden muss, eignet sich die Anordnung der Leiterplatte auf dem nahezu ebenen Handrücken: Der Handrücken stellt zudem eine harte flächige Unterlage für die Leiterplatte dar und schützt diese vor Beschädigungen. Der Handrücken ist im Wesentlichen der einzige Teil einer Hand, dessen Oberfläche bei nahezu allen Handbewegungen konstant bleibt. Die geometrischen Änderungen aufgrund der Muskel- und Knochenbewegungen sind marginal im Vergleich zu allen anderen Handoberflächen. Des Weiteren ist die Leiterplatte zusätzlich durch die Umkapselung geschützt.

Die Umkapselung ist aus Silikon hergestellt. Silikon ist vorteilhaft, da es wasserundurchlässig ist, mit vielen Materialien gut verklebt werden kann, flexibel ist und zum anderen vernähbar ist. Eine derartige Umkapselung ist gut verarbeitbar und kann auf vorteilhafte Weise später in den Handschuh integriert werden.

Bei einer weiteren Variante ist die Umkapselung aus einem oberen und einem unteren Teil geschaffen, wobei die Kammer zwischen dem oberen und unteren Teil gebildet ist und der obere und der untere Teil an ihrem jeweiligen Rand miteinander verbunden sind, vorzugsweise verklebt oder verschweißt sind, und so den flanschförmigen Rand der Umkapselung bilden. Auf diese Weise kann die Leiterplatte besonders einfach in die Umkapselung eingesetzt werden und die Umkapselung erst anschließend verschlossen werden. Dies vereinfacht die Herstellung des Bedienfelds.

In einer weiteren Variante der Heizvorrichtung weist die Umkapselung mindestens eine Öffnung zur Durchführung einer Verbindung zwischen der Leiterplatte und dem mindestens einen Wärmeübertragungselement und/oder dem mindestens einen Anschluss auf. Die Öffnung ist dabei so gewählt, dass diese erst im flanschförmigen Randbereich der Umkapselung nach außen führt. Auf diese Weise wird verhindert, dass die Kammer selbst eine Öffnung aufweist, durch welche Feuchtigkeit direkt an die Leiterplatte gelangen kann. Dies verbessert die Funktionsweise der Heizvorrichtung, da Feuchtigkeit nicht länger an die Leiterplatte gelangen kann. Dies ist möglich, da der Rand entweder eine Dicke aufweist, welche dicker ist als die Breite oder Dicke einer Verbindung zwischen der Leiterplatte und dem mindestens einen Wärmübertragungselement und/oder dem mindestens einen Anschluss oder die Öffnung zwischen einem oberen und einem unteren Teil der Umkapselung im Randbereich verläuft.

In einer weiteren Variante weist die Umkapselung Abstützelemente auf, welche an der Innenseite hervorstehen und mit der Leiterplatte verbunden sind. Über die Abstützelemente ist es möglich, einen Abstand zwischen der Umkapselung und den elektrischen und elektronischen Bauteilen auf der Leiterplatte im Regelfall zu gewährleisten. Hierbei ist der Regelfall dahingehend zu verstehen, dass keine Kraft von außen auf die Kammer einwirkt. Die Abstützelemente tragen außerdem dazu bei, dass die Umkapselung nicht vollständig auf die Leiterplatte gedrückt werden kann.

In einer weiteren Variante sind auf der Außenseite der Umkapselung mindestens zwei Bedienungselemente angeordnet, welche über den mindestens zwei auf der Innenseite der Umkapselung angeordneten Kontaktelementen liegen, so dass ein auf eines der zwei Bedienungselemente aufgebrachter Druck den darunter liegenden Schalter betätigt. Bei den Bedienelementen kann es sich beispielsweise um Markierungen oder profilierte Erhebungen der Umkapselung handeln, welche diese als Bedienelemente kennzeichnen. Der Vorteil ist eine einfachere Bedienbarkeit für den Träger der Heizvorrichtung.

In einer weiteren Variante weisen die mindestens zwei auf der Innenseite der Umkapselung angeordneten Kontaktelemente leitfähiges Material, z.B. leitfähigen Kunststoff auf. Sind die angeordneten Kontaktelemente in einer Ruhestellung, sind diese beabstandet zu den Kontaktelementen der Leiterplatte. In einer Arbeitsstellung berühren die Kontaktelemente der Umkapselung die Kontaktelemente der Leiterplatte und schließen so einen elektrischen Schaltkreis. Dadurch, dass die Kontaktelemente der Umkapselung aus leitfähigem Kunststoff bestehen können diese zum einen den Stromkreis schließen und sind zum anderen komprimierbar und druckunempfindlich, was eine verbesserte Lebensdauer des Bedienfeldes und der auf der Leiterplatte befindlichen Elektronik sicherstellen.

Bei einer weiteren Variante weisen die mindestens zwei auf der Leiterplatte ausgebildeten Kontaktelemente jeweils zwei voneinander beabstandeten, vorzugsweise kammförmig ineinander greifenden, Leiterbahnen auf. Auf diese Weise wird eine große Kontaktfläche für die Kontaktelemente der Innenseite der Umkapselung gebildet, so dass eine robustere Funktionsweise des Schalters garantiert wird.

In einer weiteren Variante ist der Steuerschaltung eine Zeitsteuerung beigefügt, wobei die Zeitsteuerung zumindest jeweils eine Zeitdauer einer Betätigung der mindestens zwei Schalter erfasst. Mittels der Zeitsteuerung kann sichergestellt werden, dass zufällige Betätigungen der Schalter zu keiner Veränderung der Temperatur der Wärmeübertragungselemente führen. Zufällige Betätigungen der Schalter sind beispielsweise beim Sport nicht selten. Die Zeitsteuerung misst die Zeit, über welche ein Schalter geschlossen bzw. gedrückt ist, und gibt erst nach einer innerhalb der Zeitsteuerung festgelegten Zeit ein Signal an die Steuerschaltung, dass eine Veränderung der eingeregelten Temperatur eingeleitet werden soll. Gleiches gilt beispielsweise für das An- und Abschalten der Heizvorrichtung. Hier kann über ein weiteres Zeitintervall, welches vorzugsweise länger als das Zeitintervall einer Veränderung der Temperatur ist, vorgesehen werden, so dass die Steuerschaltung die Heizvorrichtung aus- oder einschaltet.

In einer weiteren Variante ist auf der Leiterplatte ein Anzeigeelement angeordnet und die Umkapselung ist im Bereich des Anzeigeelements transparent, so dass eine Anzeige des Anzeigeelements außerhalb der Umkapselung für den Träger der Heizvorrichtung sichtbar ist. Über das Anzeigeelement kann ein Träger bzw. Bediener der Heizvorrichtung erkennen, welche Temperatureinstellung momentan eingestellt ist. Bei einer Änderung der Temperatur aufgrund einer Bedienung der Bedienelemente ändert sich die Anzeige entsprechend. Die Anzeige ist dabei aufgrund der Transparenz der Umkapselung im Bereich des Anzeigeelements sichtbar, jedoch vor Schmutzeinwirkungen und Fremdkörpern aufgrund der Umkapselung geschützt.

Die vorgenannten Varianten der Heizvorrichtung sind beliebig miteinander kombinierbar.

Bei einer Variante des Handschuhs weist dieser mindestens eine Tasche auf, wobei die Tasche zur Aufnahme mindestens einer, vorzugsweise aufladbaren, Batterie ausgebildet ist und der Anschluss von der Umkapselung zur Tasche innerhalb der Außenhülle geführt ist. Der Anschluss verläuft somit auf der Innenseite der Außenhülle und kann zusätzlich mit dieser vernäht werden. In der Tasche ist lediglich der Anschluss und eine kurze Verbindung offen liegend, so dass eine gewisse Flexibilität in der Bewegung des Anschlusses vorhanden ist. Die Tasche kann dabei im Bereich des Handgelenks untergebracht sein.

Eine Variante der Tasche ist dadurch gekennzeichnet, dass die Tasche durch einen wasserdichten Reißverschluss verschließbar ist und vorzugsweise mindestens zwei separat verschließbare Fächer aufweist. In den zwei, vorzugsweise mit einem Klettverschluss, verschließbaren Taschen, kann jeweils eine Batterie eingebracht werden. Diese wird über einen Batterieanschluss mit dem Anschluss der Heizvorrichtung verbunden, wobei die Anschlüsse mit Hilfe des wasserdichten Reißverschlusses vor Feuchtigkeit geschützt wird. Bei einem Batteriewechsel kann der Reißverschluss geöffnet werden und nach Öffnung der Taschen die Batterien herausgenommen und entsorgt oder wieder aufgeladen werden. Dies hat weiteren den Vorteil, dass die Heizvorrichtung im Wesentlichen unverändert innerhalb des Handschuhs angebracht ist und lediglich die Batterien bewegt werden müssen. Auch dies erhöht die Lebensdauer des Handschuhs.

Ein derartiger Handschuh wird bevorzugt hergestellt, indem zuerst die Heizvorrichtung hergestellt wird. Anschließend wird der flanschförmige Rand der Umkapselung mit einem Handrückenteil der Außenhülle verbunden. Dies kann entweder mittels Kleben oder Nähen geschehen, wobei das Handrückenteil hierbei eine Aussparung für die Bedienelemente aufweisen kann oder durchgehend sein kann. Anschließend wird das zumindest eine Wärmeübertragungselement in mindestens einer in einem der Finger eines Handschuhs befindlichen Tasche vor einer Innenseite der Außenhülle angeordnet. Des Weiteren wird der Anschluss Richtung Handgelenk verlegt. Anschließend an diese Schritte wird das Handrückenteil mit der restlichen Außenhülle des Handschuhs vernäht. Zu diesem Zeitpunkt ist die Heizvorrichtung jedoch bereits im Innenhandschuh oder der Außenhülle des Handschuhs korrekt positioniert.

Eine Heizvorrichtung und ein Kleidungsstück, beispielhaft ein Handschuh mit Heizvorrichtung, sollen anhand von einigen Ausführungsbeispielen näher erläutert werden. Es zeigen:
- Fig. 1: eine Heizvorrichtung;
- Fig. 2: einen Handschuh mit Heizvorrichtung;
- Fig. 3: einen Längsschnitt durch das Bedienfeld einer Heizvorrichtung;
- Fign. 4a und 4b: Querschnitte durch die Heizvorrichtung.

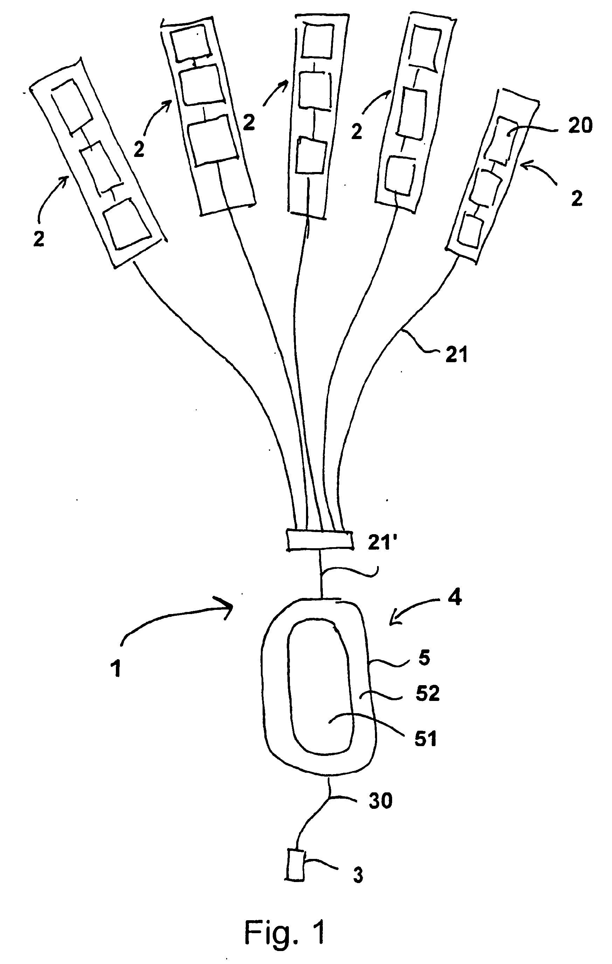

Fig. 1 zeigt eine Heizvorrichtung 1 mit fünf Wärmeübertragungselementen 2, wobei diese jeweils mindestens ein elektrisches Heizelement 20 aufweisen. Jedes Wärmeübertragungselement 2 weist eine Verbindung 21 auf, welche zu einer gemeinsamen Verbindung 21' verbunden wird. Die Verbindungen sind hierbei über elektrisch leitende Kabel mit einer Isolierschicht ausgebildet. Weiterhin ist ein Anschluss 3 für eine Energieversorgungseinrichtung vorhanden, welche ebenfalls über eine Verbindung 30 in Form eines Kabels verfügt. Sowohl die Verbindung 21' als auch die Verbindung 30 laufen in das Bedienfeld 4 ein, bei welchem in der Fig. 1 lediglich die Umkapselung 5 mit dem hervorstehenden flanschförmigen Rand 52 und der angedeuteten Kammer 51 gezeigt ist. Auf die Umkapselung 5 und die darin befindliche Leiterplatte wird in den nachfolgenden Figuren genauer eingegangen.

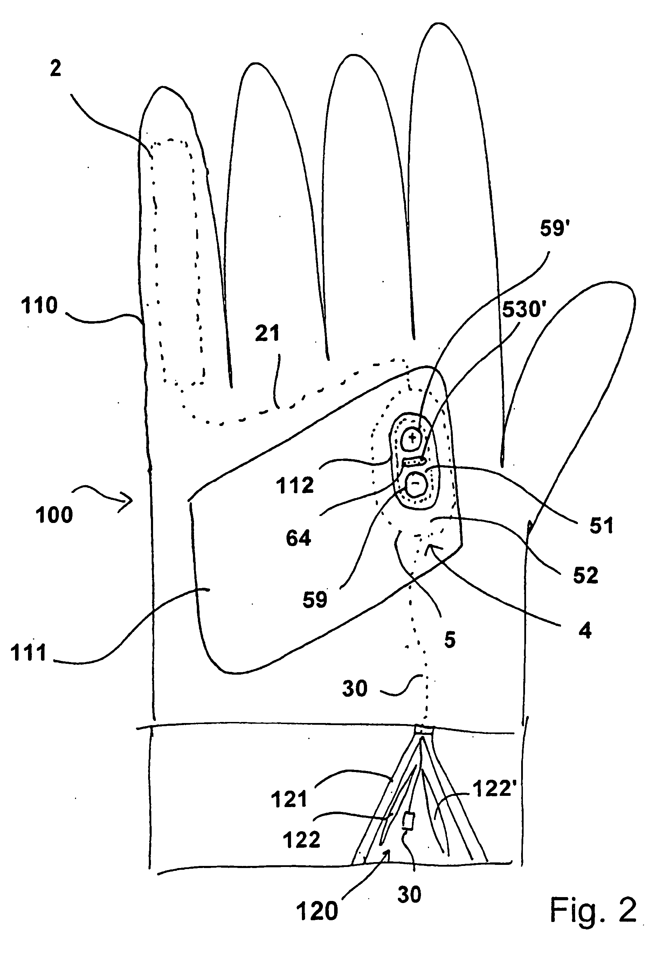

In der Fig. 2 ist ein Handschuh 100 für eine linke Hand gezeigt, in welchem eine Variante der Heizvorrichtung 1 integriert ist. Der Handschuh 100 weist eine Außenhülle 110 auf, wobei ein Teil der Außenhülle 110 durch das Handrückenteil 111 gebildet ist. In das Handrückenteil 111 ist das Bedienteil 4 eingelassen. Dabei deuten die schraffierten Linien an, dass die damit gekennzeichneten Merkmale innerhalb der Außenhülle 110 verlaufen, d.h. für einen Betrachter nach außen - außer bei einer transparenten Außenhülle - nicht sichtbar sind. Das Bedienteil 4 ist mit dem Handrückenteil 111 durch eine Naht 112 verbunden. Dabei verläuft die Naht im flanschförmigen Rand 52 der gezeigten Umkapselung 5. Die Nähte verlaufen damit um die in der Kammer 51 eingebrachte Leiterplatte umher und berühren diese nicht. Für einen Träger des Handschuhs 100 sind vom Bedienfeld 4 lediglich die Bedienelemente 59, 59' und das Anzeigefeld 64 sichtbar, da die Umkapselung 5 im Bereich 530' transparent ausgebildet ist. Die Oberfläche des Bedienfelds, welche innerhalb der Naht 112 verläuft, ist außer dem transparenten Bereich 530' nicht transparent ausgeführt.

Der Handschuh 100 weist zudem eine Tasche 120 mit einem wasserdichten Reißverschluss 121 auf. In der Tasche 120 sind zwei Fächer 122, 122' angeordnet, wobei diese mit einem Klettverschluss zumindest teilweise verschlossen werden können. Des Weiteren ist der Anschluss 30 sichtbar, welcher über die Verbindung 30 mit dem Bedienfeld 4 verbunden ist.

Eine Batterie wird über einen Batterieanschluss mit dem Anschluss 30 steckbar verbunden und versorgt das Bedienfeld und die Wärmeübertragungselemente mit Strom. Der Reißverschluss 121 wird geschlossen, so dass die Batterie und der Anschluss 30 geschützt sind. Für den Fall, dass eine aufladbare Batterie benutzt wird, wie beispielsweise ein Lithium Ionen Akkumulator, wird der Reißverschluss 121 geöffnet, der Batterieanschluss vom Anschluss 30 abgetrennt und der Batterieanschluss mit einem Ladegerät verbunden, wobei der Ladezustand an einem Ladegerät angezeigt wird. Bei der Verbindung zwischen dem Anschluss 30 und dem Batterieanschluss ist es vorteilhaft, wenn die Anschlüsse unverwechselbar verbindbar sind, um eine fehlerhaft Polung zu vermeiden. Die Unverwechselbarkeit ist dabei über am Anschluss 30 und am Batterieanschluss angeordnete Plastikschienen realisierbar.

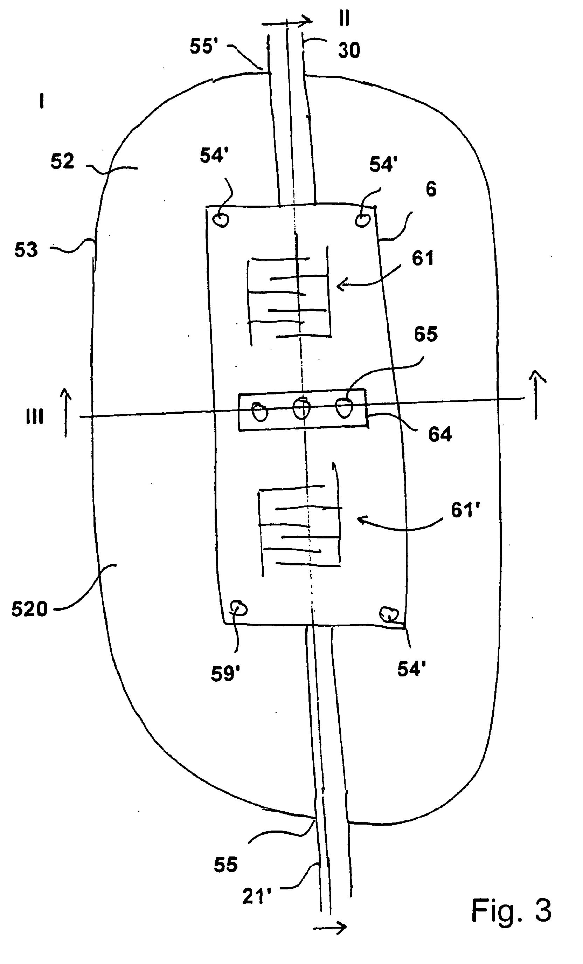

In der Fig. 3 ist das Bedienfeld 4 gezeigt, wobei die Umkapselung 5, welche beispielsweise in der Fig. 1 sichtbar ist, in Zeichenebene aufgeschnitten ist. Die Ebene ist Schnitt I genannt. Gezeigt ist ein unterer Teil 53 der Umkapselung 5, bei welcher der flanschförmige Rand 52 des unteren Teils sichtbar ist und die Kammer 51 durch die Leiterplatte 6 verdeckt ist. Auf dem Rand 520 der unteren Hälfte ist die Verbindung 30 und die Verbindung 21' gezeigt, wie diese in der Fig. 1 gezeigt waren. Auf der Leiterplatte 6 sind zudem Abstützelemente 54 gezeigt, welche in den späteren Figuren noch genauer erläutert werden.

Die Leiterplatte 6 ist eine doppelseitige Leiterplatte mit Leiterbahnen und elektrischen bzw. elektronischen Bauteilen auf beiden Seiten der Leiterplatte. Auf der hier gezeigten Seite sind Kontaktelemente 61, 61' sowie ein Anzeigefeld 64 gezeigt. Die Kontaktelemente 61, 61' weisen dabei zwei voneinander beabstandete Leiterbahnen auf, wobei die Leiterbahnen jeweils kammförmig ineinandergreifen und voneinander beabstandet sind. Das Anzeigeelement 64 weist drei Leuchtelemente 65 auf, wobei das Anzeigeelement 64 mit einer Steuerschaltung verbunden ist. In Abhängigkeit von der eingestellten Regelung leuchten so jeweils ein, zwei, drei oder keins der Leuchtelemente 65 und zeigen somit die eingestellte Regelstufe der Heizvorrichtung bzw. deren Einschalt- bzw. Ausschaltstatus an. Als Leuchtelemente sind beispielsweise ein- oder mehrfarbige LEDs geeignet.

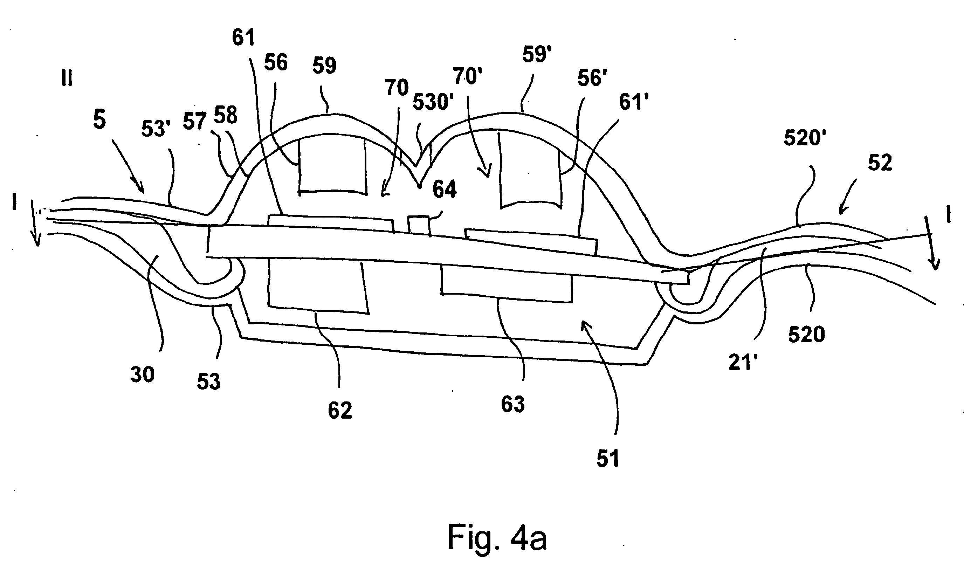

In der Fig. 4a ist das in der Fig. 3 gezeigte Bedienfeld 4 im Schnitt II gezeigt. Es ist deutlich erkennbar, dass der untere Teil 53 in Verbindung mit dem oberen Teil 53' eine geschlossene Umkapselung 5 für die Leiterplatte 6 bilden. Dabei sind lediglich Öffnungen für die Verbindungen 21' und 30 vorhanden, welche zwischen dem Rand 520 des unteren Teils und dem Rand 520' des oberen Teils hindurch verlaufen. Die Verbindung 21' weist zwischen dem Rand 520 und dem Rand 520' eine Dicke von etwa 2 mm auf. Der untere Teil 53 sowie der obere Teil 53' weisen eine Dicke zwischen 1 und 3 mm auf. Weiterhin ist der Verlauf der Schnittebene I eingezeichnet.

Die Verbindungen 21' und 30 sind mit der Leiterplatte 6 verbunden. Auf der dem oberen Teil 53' zugewandten Seite der Leiterplatte sind die Kontaktelemente 61, 61' sowie das Anzeigefeld 64 aufgebracht. Weiterhin sind auf der Außenseite 57 des oberen Teils 53' die Bedienelemente 59, 59' gezeigt, welche durch domartige Auswölbungen realisiert sind. Die Bedienelemente. 59, 59' liegen oberhalb der Schalter 70, 70', welche die Kontaktelemente 56, 56' und die Kontaktelemente 61, 61' aufweisen. Auf der Innenseite 58 der Umkapselung sind Kontaktelemente 56, 56' angebracht, wobei diese auf den den Kontaktelementen 61, 61' zugewandten Oberfläche, aus einem leitfähigen Kunststoff bestehen. Da die Oberfläche in der in Fig. 4a gezeigten Ruhestellung von den Kontaktelementen 61, 61' beabstandet sind, kann eine Veränderung des Zustands der Steuerschaltung 62 erst dann stattfinden, wenn die Kontaktelemente 56 bzw. 56' die Kontaktelemente 61, 61' berühren, und somit die kammförmig ineinandergreifenden Leiterbahnen elektrisch leitend miteinander verbinden.

Auf der Unterseite der Leiterplatte sind eine elektronische Steuerschaltung 62 sowie eine Zeitsteuerung 63 gezeigt. Die Zeitsteuerung 63 misst hierbei die Dauer des Kontakts zwischen den Kontaktelementen 56 bzw. 56' und Kontaktelementen 61 bzw. 61' und gibt die Information, dass die Schalter 70 bzw. 70' geschlossen sind, erst dann an die Steuerschaltung weiter, wenn diese für ein Zeitintervall T geschlossen waren. Hierbei ist ein Zeitraum für das Intervall T von 1 bis 2 Sekunden für das Intervall T ausreichend. Des Weiteren übergibt die Zeitsteuerung einen weiteren Befehl an die elektronische Steuerung, wenn der Schalter 70 bzw. 70' für länger als 2 bis 5 Sekunden geschlossen ist. Anhand dieses Befehls schaltet die elektronische Steuerschaltung 62 die Wärmeübertragungselemente 2 aus.

Des Weiteren ist der transparente Bereich 530' des oberen Teils 52' eingezeichnet, durch welchen die auf dem Anzeigefeld 64 vorhandene Anzeige für einen Betrachter des Bedienfeldes sichtbar ist.

In der Darstellung der Fig. 4a ist deutlich erkennbar, die die zwischen dem oberen Teil 53' und dem unteren Teil 53 gebildete Kammer 51 derart ausgebildet ist, dass im Regelfall die elektronischen Bauteile, wie beispielsweise die elektronische Steuerschaltung 62, die Zeitsteuerung 63 sowie die Kontaktelemente 61, 61' und das Anzeigefeld 64 nicht durch die Umkapselung 5 berührt werden.

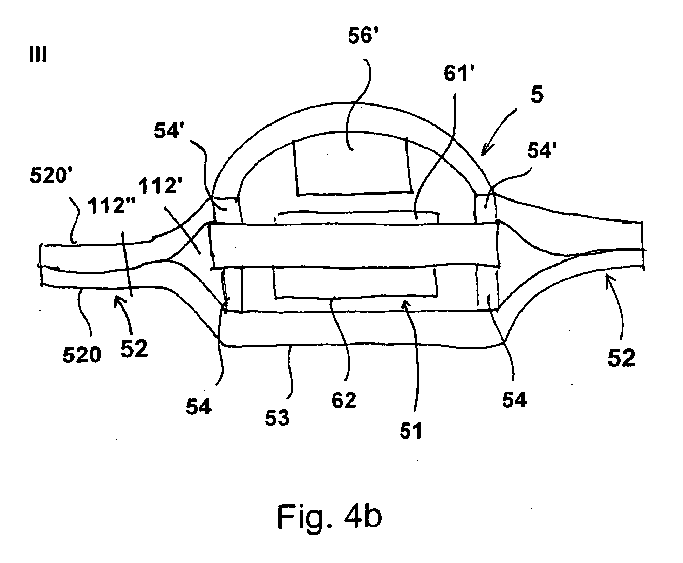

In der Fig. 4b ist der Schnitt III durch das in der Fig. 3 gezeigte Bedienfeld 4 gezeigt. Es deutlich erkennbar, wie der Rand 520 des unteren Teils 52 und der Rand 520' des oberen Teils 53' spaltfrei aufeinander liegen. Dabei sind die Ränder 520 und 520' miteinander verklebt und bilden so die geschlossene Kapsel. In der Fig. 4b sind die Abstützelemente 54 und die Abstützelemente 54' deutlich erkennbar. Sie dienen als Abstandshalter und bilden eine zusätzliche Stabilisierung der Kammer 51, so dass auch bei Aufbringen eines Drucks auf das untere Teil 53 die elektrischen Bauteile, wie beispielsweise die Steuerschaltung 62, weiterhin, zumindest teilweise, abgestützt werden. Selbiges gilt für die Abstützelemente 54' des oberen Teils 53', wobei hier eine Kontaktierung zwischen den Kontaktelementen 56' und 61' erwünscht ist.

Weiterhin sind zwei mögliche Positionen für ein Vernähen mit dem Handrückenteil eines Handschuhs eingezeichnet. Dies betrifft zum einen die Position 112', welche lediglich durch den oberen Teil der Umkapselung 5 tritt, und das Handrückenteil lediglich mit dem oberen Teil 53' vernäht, sowie zum anderen die Position 112", bei welcher eine Vernähung durch sowohl das obere Teil 53' als auch das untere Teil 53 stattfindet.

Der flanschförmige Rand kann eine Breite von 3 mm bis 2 cm, vorzugsweise von 5 mm bis 1,2 cm, aufweisen.

## Patentansprüche

1. Heizvorrichtung zum Erwärmen und/oder Temperieren einer Hautoberfläche mit Tiefenwirkung auf Teilbereiche eines menschlichen Körpers,
mit mindestens einem Wärmeübertragungselement (2), mindestens einem Anschluss (30) für eine Energieversorgungseinrichtung, mindestens einem Bedienfeld (4) mit einer Umkapselung (5) und mindestens einer Leiterplatte (6) mit einer elektrischen Steuerschaltung (62) zur Regelung der Temperatur des Wärmeübertragungselements (2) und zwei Schaltern (70,70') zum manuellen Einstellen der Temperatur, wobei die Leiterplatte (6) mit dem mindestens einen Wärmeübertragungselement (2) und dem mindestens einen Anschluss (3) verbunden ist,
**dadurch gekennzeichnet, dass** die Umkapselung (5) aus Silikon hergestellt ist und eine die Leiterplatte (6) aufnehmende Kammer (51) und einen die Kammer umgebenden flanschförmigen Rand (52) aufweist, wobei der Rand (52) aus einem flexiblem Material besteht, und die Schalter (70,70') durch mindestens zwei auf einer Innenseite (58) der Umkapselung (5) angeordnete Kontaktelemente (56,56') und mindestens zwei auf der Leiterptatte (6) ausgebitdete Kontaktelemente (61,61') jeweils einen Schalter bildend, gebildet werden und der flanschförmige Rand mit einer Außenhülle eines Handschuhs verbindbar ist.

2. Heizvorrichtung nach Anspruch 1, **dadurch gekennzeichnet, dass** die Umkapselung (5) einen oberen (53') und einen unteren Teil (53) umfasst, wobei die Kammer (51) zwischen dem oberen (53') und unteren Teil (53) gebildet ist und der obere (53') und der untere Teil (53) an ihrem Rand (520,520') miteinander verbunden, vorzugsweise verklebt oder verschweißt, sind und den Rand (52) der Umkapselung (5) bilden.

3. Heizvorrichtung nach einem der vorhergehenden Ansprüche, **dadurch gekennzeichnet, dass** die Umkapselung (5) mindestens eine Öffnung zur Durchführung einer Verbindung (21',30) zwischen der Leiterplatte (6) und dem mindestens einen Wärmeübertragungselement (2) und/oder dem mindestens einen Anschluss (3) aufweist.

4. Heizvorrichtung nach einem der vorhergehenden Ansprüche, **dadurch gekennzeichnet, dass** die Umkapselung (5) über an der Innenseite (58) hervorstehende Abstützelemente (54,54') mit der Leiterplatte (6) verbunden ist.

5. Heizvorrichtung nach einem der vorhergehenden Ansprüche, **dadurch gekennzeichnet, dass** auf der Außenseite (57) der Umkapselung (5) mindestens zwei Bedienungselemente (59,59') angeordnet sind, welche über den mindestens zwei auf der Innenseite (58) der Umkapselung (5) angeordneten Kontaktelementen (56,56') liegen, so dass ein auf eines der zwei Bedienungselemente (59,59') aufgebrachter Druck den darunter liegenden Schalter (70,70') betätigt.

6. Heizvorrichtung nach einem der vorhergehenden Ansprüche, **dadurch gekennzeichnet, dass** die mindestens zwei auf der Innenseite (58) der Umkapselung (5) angeordneten Kontaktelemente (56,56') leitfähiges Material, vorzugsweise leitfähigen Kunststoff aufweisen.

7. Heizvorrichtung nach einem der vorhergehenden Ansprüche, **dadurch gekennzeichnet, dass** die mindestens zwei auf der Leiterplatte (6) ausgebildeten Kontaktelemente (61,61') jeweils aus zwei voneinander beabstandeten, vorzugsweise kammförmig ineinander greifenden, Leiterbahnen gebildet sind.

8. Heizvorrichtung nach einem der vorhergehenden Ansprüche, **dadurch gekennzeichnet, dass** die Steuerschaltung (62) eine Zeitsteuerung (63) beinhaltet, wobei die Zeitsteuerung (63) zumindest jeweils eine Zeitdauer einer Betätigung der mindestens zwei Schalter (70,70') erfasst.

9. Heizvorrichtung nach einem der vorhergehenden Ansprüche, **dadurch gekennzeichnet, dass** an der Leiterplatte (6) ein Anzeigeelement (64) angeordnet ist und die Umkapselung (5) im Bereich des Anzeigeelements (64) transparent ist, so dass eine Anzeige des Anzeigeelements (64) außerhalb der Umkapselung (5) sichtbar ist.

10. Handschuh mit einer Außenhülle und einer mit der Außenhülle verbundenen Heizvorrichtung nach einem der vorhergehenden Ansprüche.

11. Handschuh (100) nach Anspruch 10, **dadurch gekennzeichnet, dass** mindestens eine Tasche (120) vorhanden ist und die Tasche (120) zur Aufnahme mindestens einer, vorzugsweise aufladbaren, Batterie ausgebildet ist und der Anschluss (3) von der Umkapselung zur Tasche geführt ist.

12. Handschuh (100) nach Anspruch 11, **dadurch gekennzeichnet, dass** die Tasche (120) durch einen wasserdichten Reißverschluss (121) verschließbar ist und vorzugsweise mindestens zwei verschließbare Fächer (122,122') aufweist.

13. Verfahren zur Herstellung eines Handschuhs, nach einem der Ansprüche 10 bis 12, welches folgende Schritte umfasst:
a) Herstellung der Heizvorrichtung (1);
b) Verbindung der Umkapselung (5) mit einem Teil, vorzugsweise einem Handrückenteil (111) der Außenhülle (110);
c) Anordnung des mindestens eine Wärmeübertragungselement (2) innerhalb der Außenhülle (110), vorzugsweise in mindestens einem Finger des Handschuhs;
d) Verbindung des Teils, vorzugsweise Handrückenteils (111) mit der restlichen Außenhülle (110) des Kleidungsstücks, vorzugsweise des Handschuhs (100).

## Claims

1. Heating device for heating and/or temperature-control of a skin surface with deep effect on partial regions of a human body,
comprising at least one heat transfer element (2), at least one terminal (30) for an energy supply device, at least one control panel (4) with an encapsulation (5) and
at least one circuit board (6) with an electric control circuit (62) for controlling the temperature of the heat transfer element (2) and
two switches (70, 70') for manual adjustment of the temperature, the circuit board (6) being connected to the at least one heat transfer element (2) and to the at least one terminal (3),
***characterised in that***
the encapsulation (5) is realised of silicone and has a chamber (51) receiving the circuit board (6) and a flange-like edge (52) surrounding the chamber, the edge (52) consisting of a flexible material, and the switches (70, 70') being formed by at least two contact elements (56, 56') which are disposed on an inside (58) of the encapsulation (5) and at least two contact elements (61, 61') which are configured on the circuit board (6) each forming a single switch, respectively, and the flange-like edge being connectable to an outer shell of a glove.

2. Heating device according to claim 1, **characterised in that** the encapsulation (5) comprises an upper (53') and a lower part (53), the chamber (51) being formed between the upper (53') and lower part (53) and the upper (53') and the lower part (53) being connected to each other at their edge (520, 520'), preferably glued or welded, and forming the edge (52) of the encapsulation (5).

3. Heating device according to one of the preceding claims, **characterised in that** the encapsulation (5) has at least one opening for guiding through a connection (21', 30) between the circuit board (6) and the at least one heat transfer element (2) and/or the at least one terminal (3).

4. Heating device according to one of the preceding claims, **characterised in that** the encapsulation (5) is connected to the circuit board (6) via support elements (54, 54') which protrude on the inside (58).

5. Heating device according to one of the preceding claims, **characterised in that** at least two operating elements (59, 59') are disposed on the outside (57) of the encapsulation (5), which operating elements are situated above the at least two contact elements (56, 56') which are disposed on the inside (58) of the encapsulation (5) so that a pressure applied on one of the two operating elements (59, 59') actuates the switch (70, 70') situated thereunder.

6. Heating device according to one of the preceding claims, **characterised in that** the at least two contact elements (56, 56') disposed on the inside (58) of the encapsulation (5) have conductive material, preferably conductive plastic material.

7. Heating device according to one of the preceding claims, **characterised in that** the at least two contact elements (61, 61') configured on the circuit board (6) are formed respectively from two strip conductors which are at a spacing from each other and engage in each other preferably like a comb.

8. Heating device according to one of the preceding claims, **characterised in that** the control circuit (62) includes a time control means (63), the time control means (63) detecting at least respectively a time duration of one actuation of the at least two switches (70, 70').

9. Heating device according to one of the preceding claims, **characterised in that** a display element (64) is disposed on the circuit board (6) and the encapsulation (5) in the region of the display element (64) is transparent so that a display of the display element (64) is visible outwith the encapsulation (5).

10. Glove comprising an outer shell and a heating device being connected to the outer shell according to one of the preceding claims.

11. Glove (100) according to claim 10, **characterised in that** at least one pocket (120) is present and the pocket (120) is configured for receiving at least one, preferably rechargeable, battery and the terminal (3) is guided from the encapsulation to the pocket.

12. Glove (100) according to claim 11, **characterised in that** the pocket (120) can be closed by a water-tight zip (121) and preferably has at least two closable compartments (122, 122').

13. Method for the production of a glove, according to one of the claims 10 to 12, which comprises the following steps:
a) production of the heating device (1);
b) connection of the encapsulation (5) to a part, preferably a back of the hand part (111) of the outer casing (110);
c) arrangement of the at least one heat transfer element (2) within the outer casing (110), preferably in at least one finger of the glove;
d) connection of the part, preferably back of the hand part (111) to the remaining outer casing (110) of the garment, preferably of the glove (100).

## Revendications

1. Dispositif de chauffage, pour chauffer et/ou maintenir à température une surface de peau, avec effet en profondeur sur des zones partielles d'un corps humain,
comportant au moins un élément thermoconducteur (2), au moins un point de raccordement (30) pour un dispositif d'alimentation en énergie, au moins un panneau de commande (4) muni d'un boîtier (5), et au moins une carte imprimée (6) munie d'un circuit électrique de commande (62) destiné à réguler la température de l'élément thermoconducteur (2), et deux commutateurs (70, 70') destinés au réglage manuel de la température, la carte imprimée (6) étant reliée au ou à chaque élément thermoconducteur (2) et au ou à chaque point de raccordement (3),
**caractérisé en ce que** le boîtier (5) est fabriqué en silicone, et comprend une chambre (51) logeant la carte imprimée (6), et un bord (52) en forme de collerette, entourant la chambre, le bord (52) étant constitué d'un matériau souple, et les commutateurs (70, 70') formant chacun un commutateur grâce à au moins deux éléments de contact (56, 56') disposés sur la face interne (58) du boîtier (5), et à au moins deux éléments de contact (61, 61') configurés sur la carte imprimée (6), le bord en forme de collerette étant adapté pour être relié à une enveloppe extérieure d'un gant.

2. Dispositif de chauffage selon la revendication 1, **caractérisé en ce que** le boîtier (5) comprend une partie supérieure (53') et une partie inférieure (53), la chambre (51) étant formée entre la partie supérieure (53') et la partie inférieure (53), la partie supérieure (53') et la partie inférieure (53) étant reliées l'une à l'autre au niveau de leur bord (520, 520'), de préférence par collage ou soudage, et formant le bord (52) du boîtier (5).

3. Dispositif de chauffage selon l'une des revendications précédentes, **caractérisé en ce que** le boîtier (5) comprend au moins une ouverture pour le passage d'une liaison (21', 30) entre la carte imprimée (6) et le ou chaque élément thermoconducteur (2) et/ou le ou chaque point de raccordement (3).

4. Dispositif de chauffage selon l'une des revendications précédentes, **caractérisé en ce que** le boîtier (5) est, par l'intermédiaire d'éléments d'appui (54, 54') en saillie par rapport à la face interne (58), relié à la carte imprimée (6).

5. Dispositif de chauffage selon l'une des revendications précédentes, **caractérisé en ce qu'**au moins deux éléments de commande (59, 59') sont disposés sur la face externe (57) du boîtier (5), ces éléments de commande se trouvant au-dessus des au moins deux éléments de contact (56, 56') disposés sur la face interne (58) du boîtier (5), de façon qu'une pression appliquée sur l'un des deux éléments de commande (59, 59') actionne le commutateur (70, 70') situé en-dessous.

6. Dispositif de chauffage selon l'une des revendications précédentes, **caractérisé en ce que** les au moins deux éléments de contact (56, 56') disposés sur la face interne (58) du boîtier (5) comportent un matériau conducteur, de préférence un matériau plastique conducteur.

7. Dispositif de chauffage selon l'une des revendications précédentes, **caractérisé en ce que** chacun des au moins deux éléments de contact (61, 61') configurés sur la carte imprimée (6) est formé à partir de deux pistes conductrices situées à distance l'une de l'autre, de préférence s'engrenant l'une dans l'autre sous forme d'un peigne.

8. Dispositif de chauffage selon l'une des revendications précédentes, **caractérisé en ce que** le circuit de commande (62) contient une commande temporelle (63), la commande temporelle (63) détectant au moins une durée d'une manoeuvre de chacun des au moins deux commutateurs (70, 70').

9. Dispositif de chauffage selon l'une des revendications précédentes, **caractérisé en ce qu'**un élément d'affichage (64) est disposé contre la carte imprimée (6), et **en ce que** le boîtier (5) est transparent dans la zone de l'élément d'affichage (64) de façon qu'une indication de l'élément d'affichage (64) soit visible à l'extérieur du boîtier (5).

10. Gant comportant une enveloppe extérieure, et un dispositif de chauffage selon l'une des revendications précédentes relié à l'enveloppe extérieure

11. Gant (100) selon la revendication 10, **caractérisé par** la présence d'une poche (120), la poche (120) étant configurée de façon à recevoir au moins une pile, de préférence rechargeable, le raccordement (3) étant guidé du boîtier à la poche.

12. Gant (100) selon la revendication 11, **caractérisé en ce que** la poche (120) est adapté pour être fermée par une fermeture à glissière (121) étanche à l'eau, et comprend de préférence au moins deux compartiments refermables (122, 122').

13. Procédé de fabrication d'un gant selon l'une des revendications 10 à 12, comprenant les étapes suivantes :
a) fabrication du dispositif de chauffage (1) ;
b) liaison du boîtier (5) à une pièce, de préférence une pièce (111) correspondant au dos de la main, de l'enveloppe externe (110) ;
c) mise en place du ou des éléments thermoconducteurs (2) à l'intérieur de l'enveloppe externe (110), de préférence dans au moins un doigt du gant ;
d) liaison de la pièce, de préférence la pièce (111) correspondant au dos de la main, au reste de l'enveloppe externe (110) du vêtement, de préférence du gant (100).
